# EUROPÄISCHE PATENTSCHRIFT

(11) **EP 0 843 676 B1**
(45) Veröffentlichungstag und Bekanntmachung des Hinweises auf die Patenterteilung: **07.11.2001**
(21) Anmeldenummer: 96927638.5
(22) Anmeldetag: 31.07.1996
(51) Int. Cl.: C07D 451/10, A61K 31/46

(54) **ARZNEIMITTELVORBEREITUNGEN ENTHALTEND EIN ENANTIOMER VON IPRATROPIUMBROMID MIT VERLÄNGERTER WIRKUNGSDAUER**
PREPARATIONS CONTAINING IPRATROPIUM BROMIDE ENANTIOMER WITH A PROLONGED DURATION OF ACTION
PRE'PARATIONS COMPRENANT UN ENANTIOMERE DE BROMURE D'IPRATROPIUM A DUREE D'ACTION PROLONGEE

(30) Priorität: 01.08.1995 DE 19528145
(43) Veröffentlichungstag der Anmeldung: 27.05.1998
(73) Patentinhaber: Boehringer Ingelheim Pharma KG, 55218 Ingelheim am Rhein (DE); Boehringer Ingelheim International GmbH, 55218 Ingelheim am Rhein (DE)
(72) Erfinder: BANHOLZER, Rolf, D-70597 Stuttgart (DE); REICHL, Richard, D-55435 Gau-Algesheim (DE); DISSE, Bernd, D-55127 Mainz (DE); SPECK, Georg, D-55218 Ingelheim am Rhein (DE)
(74) Vertreter: Laudien, Dieter, Dr.
(86) Internationale Anmeldenummer: EP9603364
(87) Internationale Veröffentlichungsnummer: WO9705136

(56) Entgegenhaltungen:
- WO-A-94/13262
- DE-A- 4 140 861
- US-A- 3 505 337
- JOURNAL OF CHROMATOGRAPHY, Bd. 591, - 1992 Seiten 55-63, XP000605056 E. ARVIDSSON ET AL.: "Retention processes on alpha1-acid glycoprotein-bonded stationary phase"

## Beschreibung

Die Erfindung betrifft inhalative Arzneimittelzubereitungen mit verlängerter Wirkungsdauer, gekennzeichnet durch einen Gehalt an (endo,syn)-(-)-3-(3-Hydroxy-1-oxo-2-phenylpropoxy)-8-methyl-8-(1-methylethyl)-8-azoniabicyclo[3.2.1]octan-Salzen in einer Enantiomerenreinheit von 90-100%, wobei die Salze als Anionen Bromid, Chlorid oder Acetat enthalten.

Das Racemat der oben genannten Verbindung (als Bromid) ist unter der Bezeichnung Ipratropiumbromid als Wirkstoff in anticholinergen Arzneimitteln im Handel.

Wie nun überraschenderweise gefunden wurde, sind die Wirkungsverhältnisse von Racemat, linksdrehendem und rechtsdrehendem Enantiomeren deutlich verschieden und zeigen erhebliche, vom Normalfall entscheidend abweichende Besonderheiten. Das Eutomer (d.h. das Enantiomere mit der gewünschten bzw. angestrebten Aktivität) ist das L-(-)-Enantiomere. Mit Rezeptorbindungsstudien an CHO-HM Rezeptoren konnte ermittelt werden, daß das L-(-)-Enantiomere verglichen mit dem Racemat eine rund doppelt so hohe Affinität besitzt. Dieses Verhältnis entspricht Beobachtungen, die beim Vergleich der Wirkungen von Enantiomeren und Racematen häufiger gemacht werden.

Überraschend ist im vorliegenden Fall jedoch, daß bei der inhalativen Anwendung am (narkotisierten) Hund bei Vergleich des Eutomers mit dem Racemat im Gewichtsverhältnis 1 : 2 nicht nur eine höhere Wirkungsstärke, sondern auch eine erheblich verlängerte Wirkungsdauer beobachtet wird.

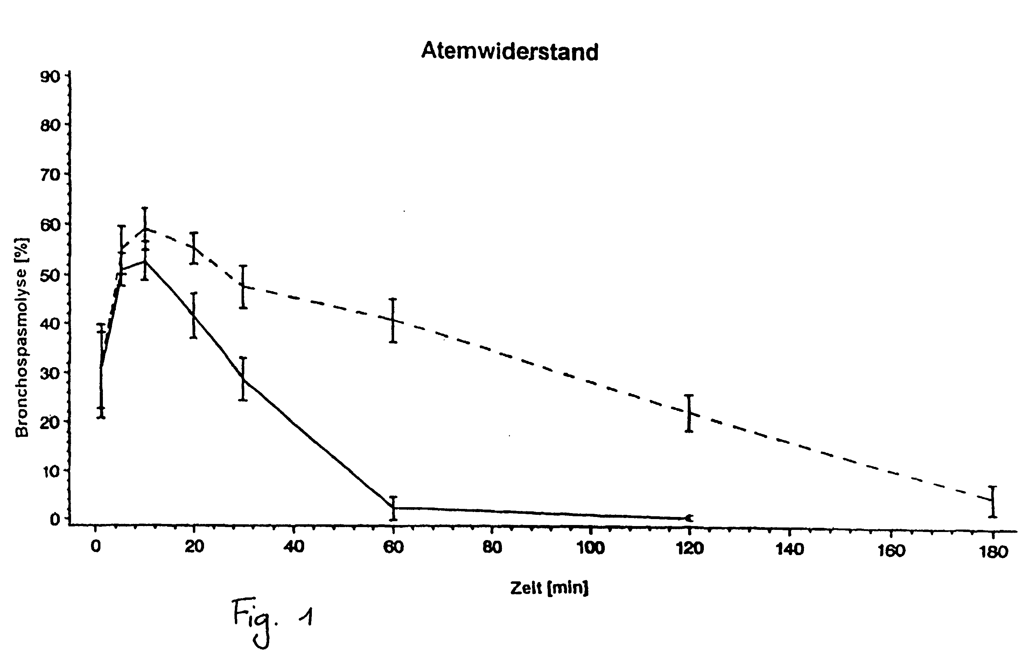

Das in Figur 1 dargestellte Schaubild gibt die prozentuale Hemmung des Bronchospasmus in Abhängigkeit von der Zeit wieder. Dabei stellt die gestrichelte Linie (Kurve B) den Verlauf für das (endo, syn)-(-)-3-(3-Hydroxy-1-oxo-2-phenylpropoxy)-8-methyl-8-(1-methylethyl)-8-azoniabicyclo[3.2.1]octan und die durchgezogene Linie (Kurve A) den Verlauf für das entsprechende Racemat dar, wobei jeweils die Hydrobromide eingesetzt wurden. Es wurden 5 µg des reinen L-(-)-Enantiomers und - entsprechend - 10 µg des Racemats verabreicht. Das Experiment wurde an fünf Versuchtieren mit dem reinen L-Enantiomeren (BIIH 150 BR) durchgeführt, während das Racemat sieben Versuchstieren appliziert wurde.

Der Figur 1 ist zu entnehmen, daß die Hemmung des durch Acetylcholin induzierten Bronchospasmus am Hund bei der Verabreichung des Ipratropiumbromid-Aerosols (Kurve A) nach etwa 10 Minuten das Maximum von ca 55 % erreicht hat und nach 60 Minuten bereits wieder Ausgangsniveau erreicht hat. Dieselbe Menge an Eutomer (Kurve 8), wie sie im Racemat enthalten ist, erreicht nach etwa 10 Minuten eine 60prozentige Hemmung, die erst nach 180 Minuten das Ausgangsniveau erreicht hat.

Anhand der gemessenen Halbwertszeiten wird für das Eutomere BIIH 150 BR eine ca. vier mal längere Wirkungsdauer festgestellt.

Zur Herstellung des Eutomers wird aus dem Racemat durch kombinierte Anwendung von Hochdruckflüssigkeitschromatographie und Umkristallisieren das Eutomer in weitgehend reiner Form gewonnen. Als "Eutomer" im Sinne der vorliegenden Erfindung werden auch stark angereicherte Produkte (über etwa 90%), vorzugsweise mit über 95, insbesondere über 97% des L-(-)-Enantiomers bezeichnet. Das Anion entspricht jeweils demjenigen in der Ausgangsverbindung. Gewünschtenfalls kann auch ein Austausch vorgenommen werden.

Durch das folgende Beispiel soll die Herstellung des L-(-)- und des D-(+)- Enantiomeren näher erläutert werden:

18 Gramm Ipratropiumbromid werden mittels Hochdruckflüssigkeitschromatographie über eine Chiralcel OD-Säule (250x20 mm) mit einer mobilen Phase der Zusammensetzung 600 Hexan, 250 Methanol, 150 Ethanol und 1 gesättigte alkoholische NaBr-Lösung (V:V:V:V:, Durchflußgeschwindigkeit 6 ml/min., Wellenlänge 254 nm; Empfindlichkeit 0,5 A.U.F.S.; Aufgabelösung 1 g Ipratropiumbromid/5 ml Ethanol + 5 ml mob. Phase + 2,5 ml konz. Essigsäure) getrennt.

Durch wiederholtes Chromatographieren und Umkristallisieren aus Ethanol werden das L-(-)-Enantiomere, weiße Kristalle, Schmp. 239-40°C (Zers.), spez. Drehung [α]²⁰_{D} = -24,06° (c = 1,014; H₂O), Enantiomerenreinheit 97,4 % (HPLC) und das D-(+)-Enantiomere, weiße Kristalle, Schmp. 238-39°C (Zers.), spezif. Drehung [α]²⁰_{D} = +24,26° (c = 1,018; H₂O), Enantiomerenreinheit 98,9 % (HPLC), erhalten. Elementaranalysen und Spektren sprechen für das Vorliegen dieser Verbindungen.

Das L-(-)-Eutomer in Form der verschiedenen Salze eignet sich, entsprechend seiner Natur als Anticholinergrkum, z.B. zur Behandlung von chronisch obstruktiver Bronchitis und Asthma durch inhalative Anwendung, wobei Nebenwirkungen weitgehend ausgeschaltet werden.

Für die Applikation wird der Wirkstoff mit bekannten Hilfs- und/oder Trägerstoffen zu gebräuchlichen galenischen Zubereitungen verarbeitet, z.B zu Inhalationslosungen. Suspensionen in verflüssigten Treibgasen, Liposomen bzw. Proliposomen enthaltenden Zubereitungen, Inhalationspulvern (gegebenenfalls in Kapseln) zur Anwendung in üblichen Inhalationsgeräten.

Formulierungsbeispiele (Angaben in Gewichtsprozent):

### 1. Dosieraerosol

| | |
|---|---|
| Wirkstoff gemäß der Erfindung | 0,005 |
| Sobitantrioleat | 0,1 |
| Monofluortrichlormethan und Difluordichlormethan 2:3 | ad 100 |

Die Suspension wird in einen üblichen Aerosolbehälter mit Dosierventil gefüllt. Pro Betätigung werden beispielsweise 50 µl Suspension abgegeben. Der Wirkstoff kann gewünschtenfalls auch höher dosiert werden (z.B. 0,02 Gew.-%).

Statt der chlorhaltigen Treibgase können auch alternative Treibgase wie TG 134a (1,1,1,2-Tetrafluorethan) und/oder TG 227 (1,1,1,2,3,3,3-Heptafluorpropan) benutzt werden.

### 2. Inhalationspulver

Mikronisiertes Wirkstoffpulver (Teilchengröße 0,5 bis 7 µm) wird mit mikronisierter Lactose gemischt und gegebenenfalls weiteren Zusätzen in Hartgelatinekapseln abgefüllt In jede Kapsel werden beispielsweise 0,01 mg Wirkstoff und 5 mg Lactose gefüllt. Das Pulver kann aus üblichen Inhalationsgeräten, z.B. gemäß DE-A 3345772, inhaliert werden.

### 3 Inhalationslösungen

Wäßrige Lösungen des Wirkstoffs konnen ebenfalls verwendet werden, wobei zum Erzeugen des Aerosols beispielsweise ein Gerät gemäß WO91/14468 benutzt werden kann. Pro Sprühvorgang werden z B 0,005 mg Wirkstoff appliziert.

Der erfindungsgemäß verwendbare Wirkstoff kann vorteilhaft auch in Kombination mit anderen Wirkstoffen für die Atemwegstherapie eingesetzt werden. Zu erwähnen sind insbesondere β₂-Mimetika, die in den Kombinationen mit 50 - 100% der Dosis bei der Einzelanwendung eingesetzt werden.
Genannt seien:
Bambuterol
Bitolterol
Carbuterol
Clenbuterol
Fenoterol
Formoterol
Hexoprenalin
Ibuterol
Pirbuterol
Procaterol
Reproterol
Salbutamol
Salmeterol
Sulfonterol
Terbutalin
Tulobuterol
1-(2-Fluor-4-hydroxyphenyl)-2-[4-(1-benzimidazolyl)-2-methyl-2-butylamino] ethanol
erythro-5'-Hydroxy-8'-(1-hydroxy-2-isopropylaminobutyl)-2H-1,4- benzoxazin-3-(4H)-on
1-(4-Amino-3-chlor-5-trifluormethylphenyl)-2-tert.-butylamino)ethanol
1-(4-Ethoxycarbonylamino-3-cyan-5-fluorphenyl)-2-(tert.-butylamino)ethanol.

Als weitere Kombinationspartner eignen sich inhalativ anwendbare Steroide wie Budesonid, Beclometason (bzw. das 17,21-Dipropionat), Dexamethason-21-isonicotinat, Flunisolid und Antiallergika wie Dinatriumcromoglicat, Nedocromil, Epinastine Auch bei diesen Kombinationspartners können gleiche oder kleinere Dosen als bei ihrer alleinigen Anwendung verabreicht werden.

## Patentansprüche

1. Inhalative Arzneimittelzubereitungen mit verlängerter Wirkungsdauer, **gekennzeichnet durch** einen Gehalt an (endo,syn)-(-)-3-(3-Hydroxy-1-oxo-2-phenylpropoxy)-8-methyl-8-(1-methylethyl)-8-azoniabicyclo[3.2.1]octan-Salzen, die als Anionen Bromid, Chlorid oder Acetat enthalten, in einer Enantiomerenreinheit von 90-100%, gegebenenfalls neben üblichen Hilfs- und/oder Trägerstoffen und/oder weiteren Wirkstoffen.

2. Verwendung von (endo,syn)-(-)-3-(3-Hydroxy-1-oxo-2-phenylpropoxy)-8-methyl-(1-methylethyl)-8-azoniabicyclo[3.2.1]octan-Salzen, die als Anionen Bromid, Chlorid oder Acetat enthalten, in einer Enantiomerenreinheit von 90 bis 100%, gegebenenfalls neben üblichen Hilfs- und/oder Trägerstoffen und/oder weiteren Wirkstoffen, zur Herstellung einer inhalativen Arzneimittelzubereitung zur Behandlung von Atemwegserkrankungen bei verlängerter Wirkungsdauer.

3. Verwendung nach Anspruch 2 zur langanhaltenden Therapie von chronisch obstruktiver Bronchitis, Bronchospasmen oder Asthma.

4. Verwendung nach Anspruch 2 oder 3, wobei als weitere Wirkstoffe β₂-Mimetika, Steroide oder Antiallergika enthalten sind.

5. Verwendung nach Anspruch 4, **dadurch gekennzeichnet, daß** als β₂-Mimetikum
Bambuterol
Bitolterol
Carbuterol
Clenbuterol
Fenoterol
Formoterol
Hexoprenalin
Ibuterol
Pirbuterol
Procaterol
Reproterol
Salbuamol
Salmeterol
Sulfonterol
Terbutalin
Tulobuterol
1-(2-Fluor-4-hydroxyphenyl)-2-[4-(1-benzimidazolyl)-2-butylamino]-ethanol erythro-5'-Hydroxy-8'-(1-hydroxy-2-isopropylaminobutyl)-2H-1,4-benzoxazin-3-(4H)-on
1-(4-Amino-3-chlor-5-trifluormethylphenyl)-2-tert.-butylamino)ethanol
1(4-Ethoxycarbonylamino-3-cyan-5-fluorphenyl)-2-(tert.-butylamino)ethanol,
als Steroid
Budesonid
Beclometason (bzw. das 17,21-Dipropionat)
Dexamethason-21 -isonicotinat
Flunisolid
und als Antiallergikum
Dinatriumcromoglicat
Nedocromil oder
Epinastin
Verwendung finden können.

6. (endo,syn)-(-)-3-(3-Hydroxy-1-oxo-2-phenylpropoxy)-8-methyl-8-(1-methylethyl)-8-azoniabicyclo[3.2.1]octan-Bromid.

## Claims

1. Inhalable pharmaceutical preparations having a prolonged period of activity, **characterised in that** they contain (endo, syn)-(-)-3-(3-hydroxy-1-oxo-2-phenylpropoxy)-8-methyl-8-(1-methylethyl)-8-azoniabicyclo[3,2,1]octane salts which contain bromide, chloride or acetate as anions, with an enantiomeric purity of 90 to 100%, optionally together with conventional excipients and/or carriers and/or other active substances.

2. Use of (endo, syn)-(-)-3-(3-hydroxy-1-oxo-2-phenylpropoxy)-8-methyl-(1-methylethyl)-8-azoniabicyclo[3,2,1]octane salts which contain bromide, chloride or acetate as anions, with an enantiomeric purity of 90 to 100%, optionally together with conventional excipients and/or carriers and/or other active substances, for preparing a pharmaceutical preparation for inhalation, for treating diseases of the respiratory tract, with a prolonged period of activity.

3. Use according to claim 2 for the long-lasting treatment of chronic obstructive bronchitis, bronchospasm or asthma.

4. Use according to claim 2 or 3, wherein β₂-mimetics, steroids or antiallergic agents are present as additional active substances.

5. Use according to claim 4, **characterised in that** the β₂-mimetic used is
bambuterol
bitolterol
carbuterol
clenbuterol
fenoterol
formoterol
hexoprenaline
ibuterol
pirbuterol
procaterol
reproterol
salbutamol
salmeterol
sulfonterol
terbutaline
tulobuterol
1-(2-fluoro-4-hydroxyphenyl)-2-[4-(1-benzimidazolyl)-2-butylamino]ethanol
erythro-5'-hydroxy-8'-(1-hydroxy-2-isopropylaminobutyl)-2H-1,4-benzoxazin-3-(4H)-one
1-(4-amino-3-chloro-5-trifluoromethylphenyl)-2-tert.-butylamino)ethanol or
1-(4-ethoxycarbonylamino-3-cyano-5-fluorophenyl)-2-(tert.-butylamino)ethanol;
the steroid used is
budesonide
beclomethasone (or the 17,21-dipropionate),
dexamethasone-21-isonicotinate, or
flunisolide;
and the antiallergic agent used is disodium cromoglycate, nedocromil or epinastine.

6. (endo, syn)-(-)-3-(3-hydroxy-1-oxo-2-phenylpropoxy)-8-methyl-8-(1-methylethyl)-8-azoniabicyclo[3,2,1]octane bromide.

## Revendications

1. Préparations médicamenteuses à inhaler à durée d'action prolongée, **caractérisées par** une teneur en sels de (endo,syn)-(-)-3-(3-hydroxy-1-oxo-2-phénylpropoxy)-8-méthyl-8-(1-méthyléthyl)-8-azoniabicyclo[3.2.1]octane, qui contiennent comme anions du bromure, chlorure ou acétate, avec une pureté énantiomérique de 90-100 %, éventuellement avec des adjuvants et/ou supports courants et/ou d'autres principes actifs.

2. Utilisation de sels de (endo,syn)-(-)-3-(3-hydroxy-1-oxo-2-phénylpropoxy)-8-méthyl-(1-méthyléthyl)-8-azoniabicyclo[3.2.1]octane, qui contiennent comme anions du bromure, chlorure ou acétate, avec une pureté énantiomérique de 90 à 100 %, éventuellement avec des adjuvants et/ou supports courants et/ou d'autres principes actifs, pour la production d'une préparation médicamenteuse à inhaler pour le traitement des maladies des voies respiratoires avec une durée d'action prolongée.

3. Utilisation selon la revendication 2 pour la thérapie de longue durée de la bronchite obstructive chronique, des bronchospasmes ou de l'asthme.

4. Utilisation selon la revendication 2 ou 3 où des β₂-mimétiques, des stéroïdes ou des antiallergiques sont contenus comme autres principes actifs.

5. Utilisation selon la revendication 4
**caractérisée en ce que**
le bambutérol,
le bitoltérol,
le carbutérol,
le clenbutérol,
le fénotérol,
le formotérol,
l'hexoprénaline,
l'ibutérol,
le pirbutérol,
le procatérol,
le reprotérol,
le salbuamol,
le salmétérol,
le sulfontérol,
la terbutaline,
le tulobutérol,
le 1-(2-fluoro-4-hydroxyphényl)-2-[4-(1-benzimidazolyl)-2-butylamino]-éthanol,
l'érythro-5'-hydroxy-8'-(1-hydroxy-2-isopropylaminobutyl)-2H-1,4-benzoxazin-3-(4H)-one,
le 1-(4-amino-3-chloro-5-trifluorométhylphényl)-2-tert. -butylamino)éthanol,
le 1-(4-éthoxycarbonylamino-3-cyano-5-fluorophényl)-2-(tert.-butylamino)éthanol, peuvent être utilisés comme β₂-mimétique,
le budésonide,
la béclométasone (ou le 17,21-dipropionate),
le 21-isonicotinate de dexaméthasone,
le flunisolide, peuvent être utilisés comme stéroïde, et
le cromoglicate de disodium,
le nédocromil ou
l'épinastine peuvent être utilisés comme antiallergique.

6. Bromure de (endo,syn)-(-)-3-(3-hydroxy-1-oxo-2-phénylpropoxy)-8-méthyl-8-(1-méthyléthyl)-8-azoniabicyclo[3.2.1]octane.
